# EUROPEAN PATENT SPECIFICATION

(11) **EP 0 278 770 B1**
(45) Date of publication and mention of the grant of the patent: **21.04.1993**
(21) Application number: 88301176.9
(22) Date of filing: 12.02.1988
(51) Int. Cl.: A61M 1/00, A61M 1/02

(54) **Autotransfusion device**
Vorrichtung für die Autotransfusion
Dispositif d'autotransfusion

(30) Priority: 13.02.1987 US 14508
(43) Date of publication of application: 17.08.1988
(73) Proprietor: SHERWOOD MEDICAL COMPANY, St. Louis, MO 63103 (US)
(72) Inventor: Ranford, Alan Bernard, St Louis Missouri 63146 (US)
(74) Representative: Porter, Graham Ronald

(56) References cited:
- EP-A- 0 092 313
- EP-A- 0 189 010
- US-A- 4 006 745
- US-A- 4 161 179
- US-A- 4 235 233

## Description

This invention relates to autotransfusion devices and more particularly to autotransfusion devices subjected to negative pressure during blood collection.

Recently, auxiliary autotransfusion blood collection containers have been employed with chest drainage units. This permits collection and reinfusion of the patients own blood thereby avoiding the use of blood from another person and the consequent possibility of transmitting a disease to the patient.

Some autotransfusion devices of this type have certain disadvantages. For example, it has been proposed to use a non-collapsible blood collection bottle upstream of a chest drainage unit so that suction is applied through the bottle to the pleural cavity of a patient with the bottle receiving drainage blood. Because the bottle is rigid it must be vented to atmosphere during reinfusion to allow the collected blood to flow from the bottle to the patient. In such a case, air is in contact with the blood and may affect its characteristics. Also, an air filter should be used to prevent air born infection from entering the bottle.

Collapsible blood collection containers have also been used in order to avoid the problems of venting rigid containers during reinfusion. However, such collapsible bag-type containers also have disadvantages. For example, during suction drainage, the collapsible bag requires apparatus to maintain the bag in an expanded condition because of the negative pressure. This has resulted in complicated bag and bag expanding devices which are expensive. In general such bags, when expanded, take on indefinite shapes and therefore have indefinite capacities; this results in inaccurate indications of the amount of blood collected.

Because drainage fluids from the pleural cavity contain solids or semi-solids, known autotransfusion devices employ a filter placed in the patient drain line. This placement of the inlet filter creates several problems in use. In most cases during the initial stages of recovery from pneumo-thoracic surgery there is some leakage of air at the wound site. This air mixes with the blood and passes through the filter producing bubbling and foaming which gradually fills the air space in the blood collection container, making it difficult to determine the amount of liquid collected. Some blood is lost when the collection bag is squeezed to expel air prior to reinfusion of the patient. Such a filter may also become blocked which can endanger the patient's life.
US-A-4 161 179 discloses a vacuum bag assembly for wound drainage which utilizes a bag of thin flexible plastic in the form of an initially flat sealed envelope having opposed walls with sealed side edges and end edges and having a sealed catheter to provide communication. Vacuum is created by a pair of springs spaced along the axis of the bag with each spring being in the form of a pair of leaf spring elements lying back-to-back with their ends in register with one another and extending from one side edge of the bag to the other, leaf spring elements being pre-stressed for bowing mutually outwardly away from the central axis. Relatively stiff flexible sheets are interposed between the springs and the walls of the bag so that upon bowing of the springs the walls of the bag are bowed outwardly uniformly over the major portion of the length thereof into distended pillow shape for development of vacuum at the catheter connection. The flexible sheets extend substantially to the side edges but are inwardly offset from the end edges so that expansion of the bag is unrestricted.

It is therefore an object of the present invention to provide an improved blood collection and reinfusion device which is collapsible, which can be maintained in a predetermined expanded condition during blood collection and which substantially overcomes the afore mentioned problems. Another object is to provide an improved autotransfusion blood collection device which can be expanded for blood collection under suction while maintaining a predetermined expanded condition and which is collapsible for reinfusion. Still another object is to provide an improved autotransfusion device adapted for connection with a chest drainage unit and which substantially avoids the disadvantages of prior art apparatus.

According to the invention there is provided a body fluid collection device comprising a bag having an inlet for receiving body fluid and opposed walls adapted for movement with stiffening means adjacent thereto, said stiffening means including relatively stiff flexible members having opposed side edges and having portions bendable outwardly on application of a squeezing force to said edges thereby to expand the bag, the members having slots extending inwardly at either end to define opposite neck portions.

The slots of such flexible members allow the members to more closely adopt the shape of the bag at the top and bottom.

Preferably the slots define a waisted portion at either end of each flexible member. In the preferred embodiment the sides of the slots diverge from the inner end and the sides of the slots are farthest apart at the mouth. In use the mouth of each slot closes up as the member assumes the shape of the expanded bag.

The bag may be formed of two flat panels seamed around the edge, one flexible member being attached to each panel on the outside.

A tubular holder may be provided to maintain the bag in the expanded condition and in the preferred embodiment the holder has a generally conical bottom surface to support the base of the bag.

The slots are generally symmetrical although those at either end of the flexible member may differ slightly according to the expanded shape of the bag.

In the preferred embodiment each slot extends toward the longitudinal centreline of the respective flexible member and the pairs of slots converge at the inner end with each other.

In a further aspect, the invention provides a blood filter in the collection bag which permits free passage of fluid from an inlet to an outlet but filters all liquid passing from the inlet to the bag. This arrangement ensures that blood clots and other debris are retained in the filter and should the filter become blocked, fluids will pass directly from the inlet to the outlet. The filter effectively prevents foam from passing to the collection bag. Gas from the patient will pass directly from bag inlet to bag outlet.

In a preferred embodiment the filter comprises an envelope of sheet material sealed around the periphery to itself and along one edge to the periphery of the bag adjacent the bag inlet and bag outlet.

Other features of the invention will be apparent from the following description of a preferred embodiment shown by way of example only in the accompanying drawings in which:-
Fig.1 is an elevation partly in section, of a chest drainage system including an autotransfusion device in accordance with a preferred embodiment of the present invention;
Fig.2 is a cross-section taken on line 2--2 of Fig.1;
Fig.3 is an enlarged side view of the bag assembly shown in Fig.1 but in its unrestrained or free state condition;
Fig.4 is an enlarged partial side view of the upper portion of the bag assembly of Fig.3 with parts broken away;
Fig.5 is an enlarged side elevation of the autotransfusion device of Fig.1 rotated 180° about the vertical axis with the holder of Fig.1 broken away and parts removed;
Fig.6 is a cross-section taken on line 6--6 of Fig.5;
Fig.7 is a cross-section taken on line 7--7 of Fig.5;
Fig.8 is a cross-section taken on line 8--8 of Fig.4;
Fig.9 is a plan view of the filter shown in Fig.1 during a stage in the manufacture of the device of Fig.1;
Fig.10 is a top view of the holder shown in Fig.1 when apart from the other apparatus; and
Fig.11 is a cross-section taken on line 11--11 of Fig.10.

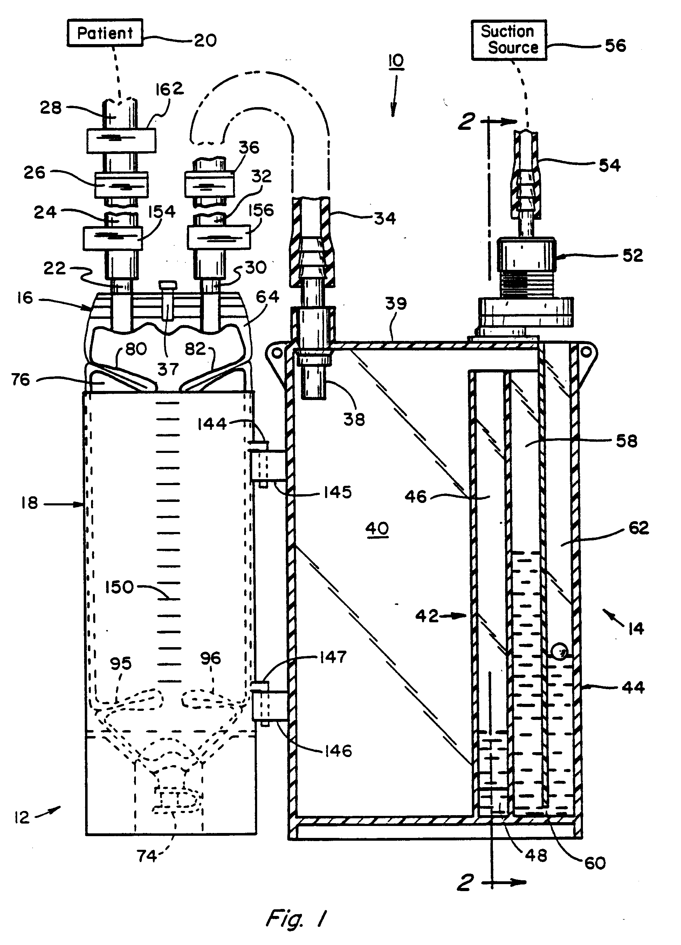

Referring now to the drawings and particularly Fig.1, a chest drainage system 10 is shown including an autotransfusion blood collection device 12 connected to the side of a chest drainage unit 14. The device 12 includes a blood collection bag assembly 16 and a bag expanding member or holder 18.Holder 18 is releasably connected to the side of the chest drainage unit 14. and as,will be discussed maintains the bag assembly 16 in an expanded condition for receiving body fluids from the plueral cavity of a patient indicated at 20.

The bag assembly 16 has an inlet 22 connected to a tube 24. Tube 24 is connected through a tube connector 26 to the proximal end of a patient tube 28 which is connected through a catheter to the pleural cavity of the patient 20. Spaced from the inlet 22 at the top of the bag assembly 16 is a gas outlet 30 communicating through the interior of the bag assembly with inlet 22. Gas outlet 30 is connected through a tube 32, tube connector 36 and tube 34 to an inlet 38 of the chest drainage unit 14. An auxiliary inlet 37 between the inlet and outlets 22 and 30 communicates with the interior of the bag and is preferably a self-sealing plug which can be penetrated by a syringe needle for introducing a substance into the bag assembly 16, for example, an anticoagulant.

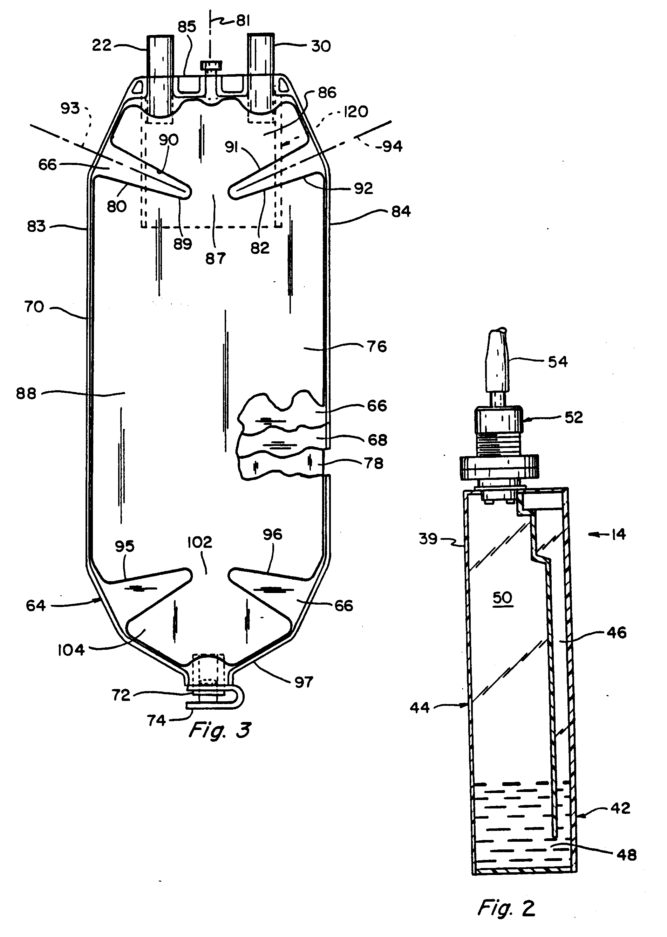

Chest drainage unit 14 is shown for illustration and includes a housing 39, preferably of rigid transparent plastic, for example, a polycarbonate or the like. The housing 39 includes a fluid collection chamber 40, an liquid seal chamber indicated generally at 42, and a liquid manometer indicated at 44. The seal chamber includes a relatively narrow vertical channel 46 open at the top where it is in fluid communication with the collection chamber 40. Channel 46 has an opening 48 at the bottom which communicates with a relatively large gas chamber 50 as seen in Fig. 2. Connected to the top of outlet chamber 50 is a suction regulator 52 that is connected by a flexible tube 54 to a suitable source of suction 56 (Fig.1). The manometer includes a vertical channel 58 in fluid communication with collection chamber 40 at the top, and, at the bottom, by a passage 60 to a vertical channel 62 that is open at the top to atmosphere. Both the seal chamber 42 and the manometer 44 are shown with liquid therein.

When the chest drainage system 10 is operating, a partial vacuum exists in the underwater seal chamber 50 (Fig. 2), and any air or gas from the patient flows from tube 28 into the upper portion of bag assembly 16 through inlet 22, then via outlet 30, into collection chamber 40.
The gas then bubbles through the liquid in seal channel 46, through the bottom opening 48 and upwardly through the chamber 50 to the suction source 56. The seal 42 prevents any atmospheric air from flowing through the unit to the patient. The manometer is responsive to the pressure in collection chamber 40, and the level of liquid in the manometer will therefore provide an indication of the negative pressure in the pleural cavity of the patient. The regulator 52 limits the negative pressure in the collection chamber 40 to a desired and safe value. The construction and operation of the chest drainage unit 14 including that of the suction regulator 52 is described in detail in U.S. No. 4,372,336.

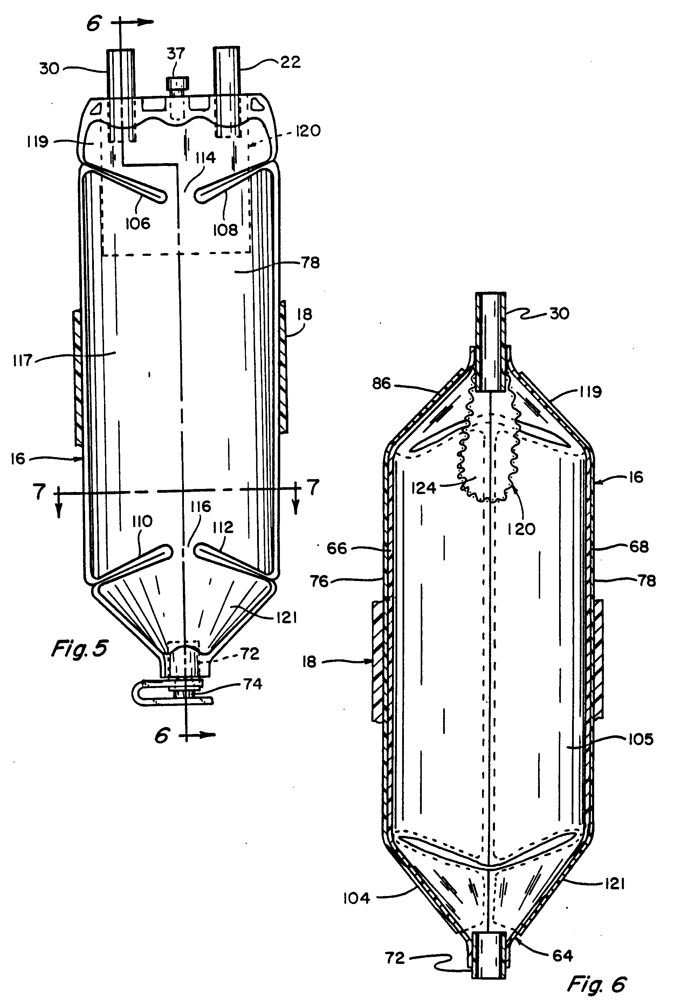

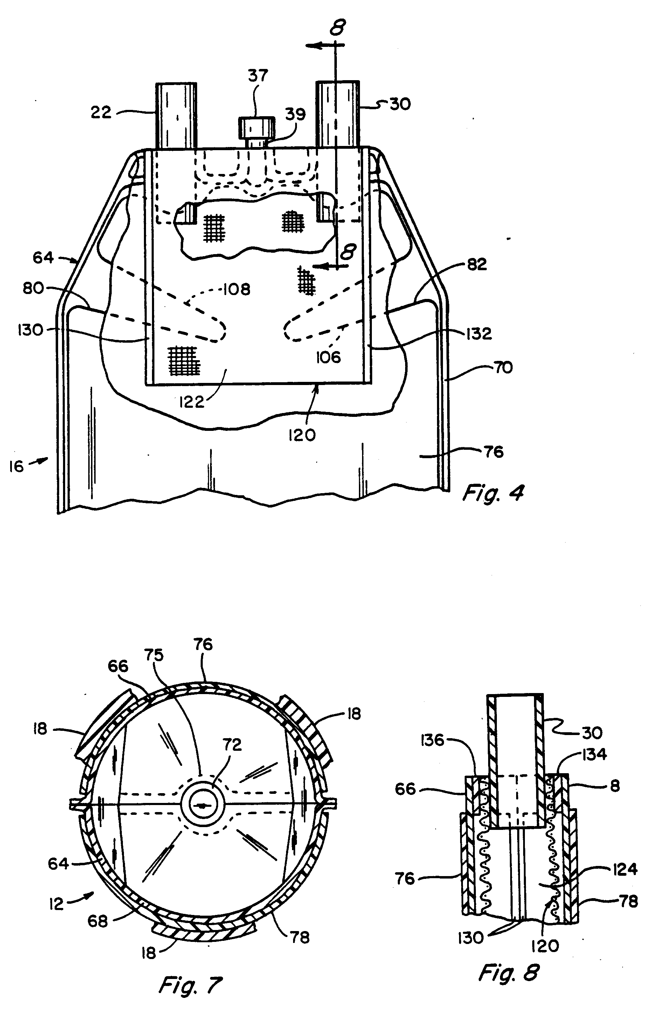

The collapsible blood collection and reinfusion bag assembly 16 is shown in Figs. 3 and 4 in its free condition so that the assembly is substantially flat. Assembly 16 includes a collapsible bag 64 formed of a pair of opposed sheet members or panels 66 and 68 seamed together about the periphery, for example by heat sealing. The peripheral seal 70 extends around the tubular inlet 22 and a gas outlet 30 and also extends around and seals a tubular blood outlet 72 at the bottom of the assembly. The blood outlet 72 is closable by a plug 74 integrally tethered to the bag 64. The blood outlet 72 shown in Fig. 7 has a conventional piercable seal 75. Plug 74 keeps the seal 75 clean. The panels 66 and 68 of bag assembly 16 are preferably formed of pliable material such as a relatively soft polyvinyl chloride so that the bag is readily and easily collapsible.

Bag assembly 16 also includes a pair of flexible bag stiffener members 76 and 78 respectively fixed to the outer surfaces of front and back panels 66 and 68 of bag 64. Stiffener members or panels 76 and 78 are in the form of generally flat sheet members of relatively stiff material, preferably, of a relatively rigid, resilient material such as polyethylene terephthalate (PET-G)sheet.

In the preferred embodiment stiffening members 76 and 78 are identical in shape and each of the panels is symmetrical about the longitudinal axis 81 as shown in Fig. 3. Panel 76 is provided with a pair of slots 80 and 82 which extend inwardly from the opposed sides of the panel and are near, but spaced from, the upper edge 85 so that a portion 86 above the slots is connected to the main body by a relatively narrow neck 87.
The slots 80 and 82 intersect the periphery of the stiffener member and have opposed sidewalls 89 and 90, and 91 and 92, respectively, the slots narrowing toward the inner ends. The longitudinal axes of the slots, indicated at 93 and 94 in Fig. 3, intersect the longitudinal axis 81 at about 68°. Panel 76 also has a similar pair of slots 95 and 96 adjacent to but spaced from the bottom edge indicated at 97, and which extend upwardly respectively from the sidewalls 83 and 84. Slots 95 and 96 also are widest at the periphery and have opposed sidewalls 98 and 99, and 100 and 101. The slots 95 and 96 also have axes which intersect the longitudinal axis 81 at about 68°. The inner ends of the slots 95 and 96 are spaced from each other to provide a relatively narrow neck 102 that connects a lower portion 104 to the main body 88. The neck portions 87 and 102 are relatively flexible and readily bend to allow the upper, main and lower portions of each stiffener member to curve inwardly to form the expanded bag assembly 16 when compressive forces are applied to the opposed sides 83 and 84 of the assembly and consequently to the opposed side edges of the stiffener member 76 and 78. The bag assembly 16 thus has an essentially cylindrical central portion, and inclined upper and lower portions when inserted into the holder 18, as seen in Figs. 5,6,7.

Stiffening member 78 is identical to member 76. Member 78, is best seen in Fig.5 and 6 where the bag assembly 16 is shown in its expanded condition in the holder 18. Member 78 has an upper pair of slots 106 and 108 which correspond to slots 80 and 82 in member 76 and a lower pair of slots 110 and 112 which correspond to slots 95 and 96. Stiffener member 78 also has upper and lower narrow neck portions 114 and 116 which define upper and lower stiffener end portions 119 and 121 respectively. The two stiffener members 76 and 78 are fixed to the bag 64 with the corresponding slots and portions of the two stiffener members in registration.

The bag contains a filter 120, best seen in Figs. 4,6,8 and 9. The illustrated filter 120 comprises a bag of fine plastic mesh defining a filter chamber 124. The inlet 22 and gas outlet 30 are sealed into the filter chamber 124.

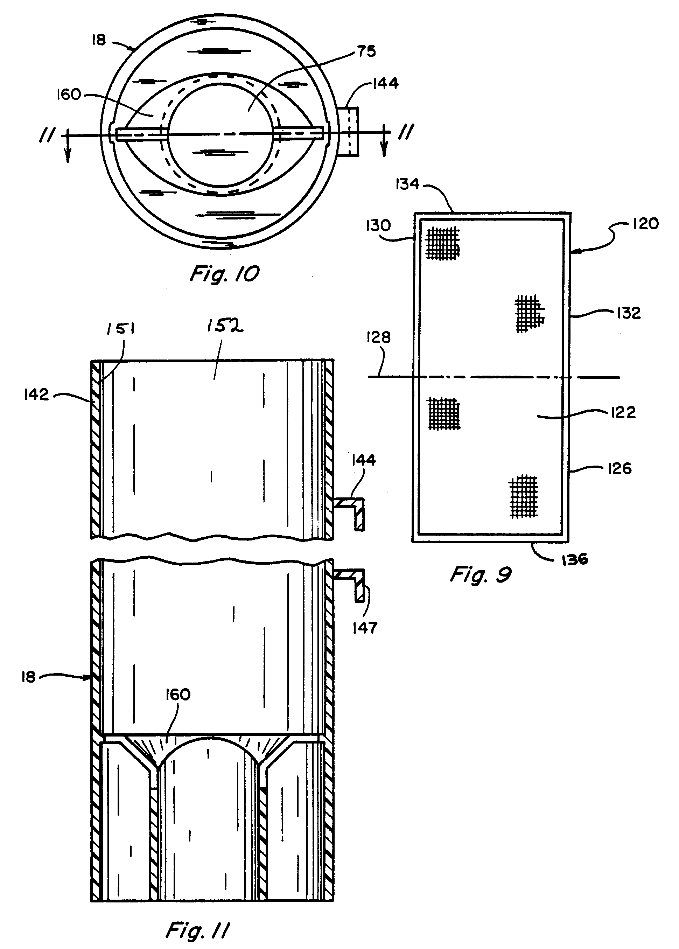

Filter 120 may be formed by cutting suitable filtering screen material 122 into rectangular shape as shown in Fig.9. Then a frame formed from sheet material, such as a thermoplastic, for example, a polyvinyl chloride, can be melt bonded to the periphery to form a border indicated at 126. The thus formed element can be folded in half, such as along the fold line 128, and the opposed sides 130 and 132 fused together. The opposed ends 134 and 136 are heat sealed together about the inlet 22, outlet 30, and auxiliary inlet 37.

After the collection bag panels 66 and 68 have the stiffener members 76 and 78 secured thereto the peripheral seal 70 is formed in order to seal the peripheries of panels 66 and 68 together and with the upper portion of the filter 120, including the ends 134 and 136 (Fig. 9),sealed to and between the panels 66 and 68 at the top end 85 of the bag assembly 16. The upper end of the filter 120 is preferably sandwiched between the panels 66 and 68.

When opposed compressive forces are applied to the opposed lateral sides 83 and 84 of the bag assembly 16 (Fig.3) the central portions of stiffener members 76 and 78 bow outwardly tending to take a generally circular cross-section and carry the walls of the bag with them. When such forces are applied, and the assembly 16 is inserted into the tubular holder 18, the bag is expanded as indicated in Figs. 1,5,6 and 7. As the opposed side edges 80 and 84 of assembly 16, including the adjacent side edges of the stiffener members 76 and 78, are moved toward each other in response to such compressive forces, the sidewalls or edges of each of the slots, 80, 82, 95, 96, 106, 108, 110 and 112 move toward each other tending to close the slot as seen in Figs. 1 and 5.

The slots being wider at the mouth near the periphery of the bag assembly 16 permit the slots to close and allow the bag to readily assume the wedge shape at the top of the assembly and generally conical shape at the bottom of the assembly. The relatively narrow neck portions 87 and 102 of stiffener member 76 and neck portions 114 and 116 of panel 78 bend to allow the upper and lower portions of the assembly 16 to incline when the bag is expanded. The stiffener members 76 and 78 are preferably connected substantially over their entire surfaces to the bag panels 66 and 68 respectively to cause the bag panels to bow outwardly with the stiffener members. The stiffener members and bag panels may be heat bonded or welded together over the entire meeting surfaces. However, a suitable adhesive could be used in some cases.

With this construction, the bag assembly 16 readily takes on the desired predetermined configuration which will be maintained at normal levels of suction so that each bag manufactured will have substantially the same predetermined internal volume during operation of the chest drainage system and such assemblies can therefore be calibrated to provide relatively acurate indications of the volume of blood collected at any time. Calibration marks are shown at 150 on the holder 18 in Fig.1 and may include graduations in centilitres.

The holder 18, as shown also in Figs. 10 and 11, includes a substantially rigid cylindrical member 142 having lugs 144 and 147 integrally connected thereto for supporting engagement with suitable receptors 145 and 146 mounted onto one side of the chest drainage unit 14 as shown in Fig.1. When desired, a releasable spring latch (not shown) may be provided on the holder 18 such as between the lugs and a cooperating latch pin(not shown) on the side of chest drainage unit 14. Such a latch and pin would ensure that the autotransfusion device 12 and unit 14 would remain together during use yet facilitate easy removal and replacement of the autotransfusion unit.

The holder 18 has a cylindrical inner wall 151 defining a cylindrical chamber 152 for receiving and maintaining the bag assembly 16 in a generally cylindrical shape. The opposed sides of the chamber, or diameter in the illustrated embodiment, are chosen according to the width of the bag assembly 16 so that the chamber walls 151 continuously exert the necessary opposed compressive forces on the opposed side edges of the stiffener members adjacent the opposed sides 83 and 84 (Fig.3) of the bag assembly 16 to maintain the bag assembly in an expanded condition as shown.

Near the bottom of the holder 18 is a generally elliptical and inclined surface 160 which provides support to the generally conical bottom of the bag assembly 16 and locates it in the holder; the bottom of the bag assembly is also somewhat elliptical in the expanded condition.

In operation, blood and gas flow from the pleural cavity of patient 20 through the inlet 22 into the interior of filter 120. Blood passes through the filter wall and flows downwardly into the blood collection chamber 105 of the bag 64 while gas and air from the inlet 22 flow under the force of suction to the gas outlet 30 without passing through the filter wall. The gas flows from outlet 30 into tube 34 and the drainage chamber 40 by way of inlet 38. Solid or semi-solid particles are held within the filter bag.

When the collected blood is subsequently reinfused into the patient such particles do not block the fine filter conventionally used in an infusion line. The filter is connected about the inlet 22 and gas outlet 30 so that both of them communicate with the interior of the filter and are in direct fluid communication with each other. Since air or gas from the inlet 22 can pass directly to outlet 30, that is, without passing through filter screen 122, the suction or negative pressure does not cause blood within the filter to mix with air and cause foaming. That is, blood is not churned by the suction force tending to produce air bubbles or foam. If foam is drained from the patient it will not pass in substantial amounts to the interior of the bag.

If the filter should become blocked, blood from the patient will flow directly through the outlet tube 30 into the collection chamber 40 thus protecting the patient by maintaining a normal suction force.

Furthermore, should the bag 64 become full, blood will overflow through tube 30 and into the collection chamber 40. It is preferable to remove the autotransfusion device 12 from the chest drainage unit 14 before the bag is full or, as would normally be the case, after a predetermined length of time.

When it is desired to infuse the patient with his own blood, tube clamps indicated at 154 and 156 and a patient tube clamp 162, which are open during fluid collection, are actuated to close tubes 24 and 28 and 32. Then the patient tube 28 and tube 34 may be disconnected from the tube connectors 26 and 36 so as to free the autotransfusion device 12 from the chest drainage unit 14. Tubes 28 and 34 are then connected together and the patient tube clamp 160 released so that patient chest drainage suction is maintained. Next, the blood collection and reinfusion bag assembly 16 can be slid upwardly and out of the holder 18 and connected to a suitable frame or the like near the patient such as by using a hanger strap that may be provided on the the unit (not shown). By momentarily releasing clamp 156 and gently squeezing the bag assembly 16, excess air can be eliminated from the bag. Next, cap 74 at the bottom of the bag assembly can be removed from the blood outlet 72 whereupon a conventional spike can be inserted through the pierceable seal 75, the spike being connected to an infusion tube or catheter via a filter and drip chamber to reinfuse the patient's own blood.

Should it be deemed necessary to collect further blood for reinfusion to the patient, the holder 18 may be disconnected from the chest drainage unit 14 and discarded to be replaced by a new bag and holder assembly 12. To reconnect, the patient tube clamp 162 must be closed, the patient tube 28 disconnected from the tube 34 and reconnected to the bag assembly inlet connector 26. Tube 34 is then connected to bag assembly outlet connector 36. Functioning is resumed by releasing patient tube clamp 160.

The stiffener members 76 and 78 of bag assembly 16, when the assembly is in the holder 18, provide the blood collection container bag of predetermined volume similar to a container having rigid sidewalls. At the same time, since the blood collection bag assembly 16 is also collapsible, it can be used in a similar way to a conventional collapsible blood collection bag. Also, a conventional well-known blood bag pressurizing sleeve can be used to squeeze the assembly 16 to thereby infuse blood at a rate faster than that accomplished by use of gravity only.

Because there is direct fluid communication between the inlet 22 and outlet 30, suction to the patient will be maintained even if the filter 120 becomes clogged. In contrast, where a filter is placed in the patient drain line upstream of the collection container, as in prior drainage systems, should such a filter become clogged, the suction to the patient would be reduced or cut-off and this would endanger the patient's life.

Preferably, the autotransfusion device 12 is assembled at the factory so that the bag assembly 16 is in the expanded condition in the holder 18 with sterile air in the bag 64. However, where desired, the bag assembly 16 could be supplied in flattened or collapsible condition if a vent with an air filter is provided.

The holder 18 and chest drainage device housing may be formed of acrylic material as well as other relatively rigid plastics. Preferably, the stiffener, holder, bag and chest drainage unit are formed of transparent plastic so that blood can be seen and monitored during operation of the system.

## Claims

1. A body fluid collection device (18) comprising a bag having an inlet (22) for receiving body fluid and opposed walls (66,68) adapted for movement with stiffening means adjacent thereto, characterised in that said stiffening means include relatively stiff flexible members (76,78) having opposed side edges and having portions bendable outwardly on application of a squeezing force to said edges thereby to expand the bag, the members (76,78) having slots (106-112) extending inwardly at either end to define opposite neck portions(119,121).

2. A device according to Claim 1 wherein the slots (106-112) define opposite waisted portions.

3. A device according to Claim 1 or Claim 2 wherein the sides of said slots (106-112) diverge from the inner end.

4. A device according to Claim 3 wherein the sides of said slots (106-112) are farthest apart at the mouth.

5. A device according to any preceding Claim wherein said flexible members (76,78) are symmetrical about the axis between the opposed side edges and the slots (106-112) are symmetrical about said axis.

6. A device according to Claim 5 wherein each of said slots (106-112) is symmetrical about its centre line.

7. A device according to any preceding Claim and including a holder (18) for maintaining a squeezing force on said edges thereby to hold the bag in an expanded condition.

8. A device according to Claim 7 wherein the holder is generally tubular.

9. A body fluid collection device as claimed in any one of the preceding Claims having a filter (120) over said inlet (22) to filter incoming liquid to the lower part of the bag and an outlet (30) adjacent said inlet and in direct fluid communication therewith whereby fluid can flow between said inlet & outlet without passing through said filter.

10. A device according to Claim 9 wherein said filter (120) comprises a sealed chamber into which said inlet (22) and outlet (30) open.

11. A device according to Claim 10 wherein said filter (120) comprises an envelope of sheet material sealed around the periphery to itself.

## Patentansprüche

1. Körperflüssigkeits-Sammelvorrichtung (18), mit einem Beutel, der einen Einlaß (22) zum Aufnehmen von Körperflüssigkeit und gegenüberliegende Wände (66, 68) aufweist, die für eine Bewegung mit dazu benachbarten Versteifungsmitteln geeignet sind, dadurch gekennzeichnet, daß die Versteifungsmittel relativ steife flexible Elemente (76, 78) enthalten, die gegenüberliegende Seitenränder haben und Teile aufweisen, die bei Anwendung einer Druckkraft auf die Ränder nach außen biegbar sind, um dadurch den Beutel aufzuweiten, wobei die Elemente (76, 78) Schlitze (106-112) haben, die sich an jedem Ende nach innen erstrecken, um gegenüberliegende Halsteile (119, 121) zu definieren.

2. Vorrichtung nach Anspruch 1, bei welcher die Schlitze (106-112) gegenüberliegende eingezogene Teile definieren.

3. Vorrichtung nach Anspruch 1 oder 2, bei welcher die Seiten der Schlitze (106-112) vom inneren Ende weg divergieren.

4. Vorrichtung nach Anspruch 3, bei welcher die Seiten der Schlitze (106-112) an der Mündung am weitesten voneinander entfernt sind.

5. Vorrichtung nach einem der vorhergehenden Ansprüche, bei welcher die flexiblen Elemente (76, 78) um die Achse zwischen den gegenüberliegenden Seitenrändern symmetrisch sind, und die Schlitze (106-112) um die Achse symmetrisch sind.

6. Vorrichtung nach Anspruch 5, bei welcher jeder der Schlitze (106-112) um seine Mittenlinie symmetrisch ist.

7. Vorrichtung nach einem der vorhergehenden Ansprüche, und mit einem Halter (18) zum Aufrechterhalten einer Druckkraft an den Rändern, um dadurch den Beutel in einem aufgeweiteten Zustand zu halten.

8. Vorrichtung nach Anspruch 7, bei welcher der Halter allgemein rohrförmig ist.

9. Körperflüssigkeits-Sammelvorrichtung nach nach einem der vorhergehenden Ansprüche, mit einem Filter (120) über dem Einlaß (22), um eintretende Flüssigkeit zum unteren Teil des Beutels zu filtern, und einem Auslaß (30) benachbart dem Einlaß und in direkter Fluidverbindung mit diesem, wodurch Fluid zwischen dem Einlaß und dem Auslaß fließen kann, ohne durch das Filter zu strömen.

10. Vorrichtung nach Anspruch 9, bei welcher das Filter (120) eine abgedichtete Kammer umfaßt, in die der Einlaß (22) und der Auslaß (30) münden.

11. Vorrichtung nach Anspruch 10, bei welcher das Filter (120) eine Umhüllung aus Folienmaterial aufweist, die rund um den Umfang in sich versiegelt ist.

## Revendications

1. Dispositif de collecte de fluide corporel (12), comprenant une poche comportant une entrée (22) destinée à recevoir le fluide corporel et des parois opposées (66, 68) propres à se déplacer avec des moyens raidisseurs adjacents, caractérisé en ce que les moyens raidisseurs comprennent des éléments flexibles relativement raides (76, 78) comportant des bords latéraux opposés et des parties pouvant fléchir vers l'extérieur lorsqu'une force de compression est exercée sur les bords de manière à déployer la poche, les éléments (76, 78) présentant des fentes (106-112) qui s'étendent vers l'intérieur, à l'une et à l'autre extrémité, afin de définir des cols opposés (119, 121).

2. Dispositif suivant la revendication 1, dans lequel les fentes (106-112) définissent des parties resserrées opposées.

3. Dispositif suivant la revendication 1 ou 2, dans lequel les côtés des fentes (106-112) divergent à partir de l'extrémité intérieure.

4. Dispositif suivant la revendication 3, dans lequel les côtés des fentes (106-112) sont écartés au maximum au niveau de l'embouchure.

5. Dispositif suivant l'une quelconque des revendications précédentes, dans lequel les éléments flexibles (76, 78) sont symétriques par rapport à l'axe entre les bords latéraux opposés et les fentes (106-112) sont symétriques par rapport à cet axe.

6. Dispositif suivant la revendication 5, dans lequel chacune des fentes (106-112) est symétrique par rapport à son axe central.

7. Dispositif suivant l'une quelconque des revendications précédentes, comprenant un support (18) pour maintenir une force de compression sur les bords afin de retenir la poche dans un état déployé.

8. Dispositif suivant la revendication 7, dans lequel le support est généralement tubulaire.

9. Dispositif de collecte de fluide corporel suivant l'une quelconque des revendications précédentes, comportant un filtre (120) sur l'entrée (22) pour filtrer le liquide entrant vers la partie inférieure de la poche et une sortie (30) adjacente à l'entrée et en communication de fluide directe avec celle-ci, de sorte que du fluide peut s'écouler entre l'entrée et la sortie sans traverser le filtre.

10. Dispositif suivant la revendication 9, dans lequel le filtre (120) comprend une chambre scellée de manière étanche, dans laquelle s'ouvrent l'entrée (22) et la sortie (30).

11. Dispositif suivant la revendication 10, dans lequel le filtre (120) comprend une enveloppe de matière en feuille scellée à elle-même de manière étanche tout autour de sa périphérie.
